(19) Europäisches Patentamt
European Patent Office
Office européen des brevets

(11) Veröffentlichungsnummer: **0 008 100**
**B1**

# (12) EUROPÄISCHE PATENTSCHRIFT

(45) Veröffentlichungstag der Patentschrift: **05.05.82**

(51) Int. Cl.³: **C 08 F 8/00,** C 08 F 212/08, C 12 N 11/08, C 07 G 7/00

(21) Anmeldenummer: **79102809.5**

(22) Anmeldetag: **04.08.79**

## (54) Verfahren zur Herstellung eines makroporösen polymeren Trägermaterials zur kovalenten Bindung von Proteinen.

(30) Priorität: **07.08.78 DE 2834539**

(43) Veröffentlichungstag der Anmeldung: **20.02.80 Patentblatt 80/4**

(45) Bekanntmachung des Hinweises auf die Patenterteilung: **05.05.82 Patentblatt 82/18**

(84) Benannte Vertragsstaaten: **AT BE CH DE FR GB IT LU NL SE**

(56) Entgegenhaltungen:
**FR-A-2 242 111**
**FR-A-2 396 030**
**GB-A-1 252 336**

(73) Patentinhaber: **BASF Aktiengesellschaft, Carl-Bosch-Strasse 38, D-6700 Ludwigshafen (DE)**

(72) Erfinder: **Tschang, Chung-Ji, Dr. Dipl.-Chem., Albrecht-Dürer-Ring 14, D-6710 Frankenthal (DE)**
Erfinder: **Klefenz, Heinrich, Dr. Dipl.-Chem., Schillerstrasse 10, D-6701 Hochdorf-Assenheim 2 (DE)**
Erfinder: **Sanner, Axel, Dr. Dipl.-Chem., Lorscher Ring 2 C, D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# Verfahren zur Herstellung eines makroporösen polymeren Trägermaterials zur kovalenten Bindung von Proteinen

Die Erfindung betrifft ein Verfahren zur Herstellung eines makroporösen polymeren Trägermaterials zur kovalenten Bindung von aktiven Proteinen über Isocyanat-, Thioisocyanat- oder Aldehydgruppen.

Proteine im Sinne der Erfindung sind biologisch aktive Substanzen, vorzugsweise Enzyme. Solche Substanzen finden u.a. Anwendung in der medizinischen Analytik, bei der Herstellung pharmazeutischer Produkte, der Herstellung optisch aktiver Substanzen sowie bei der Herstellung von Nahrungsmitteln, wie beispielsweise die Isoglucose. Die Vorteile der Verwendung von gebundenen Proteinen ergeben sich aus ihrer Wiederverwendbarkeit, ihrer einfachen Abtrennung vom Substrat bzw. dessen Lösung, ihrer oftmals erhöhten Stabilität im Vergleich zur löslichen Form, aus der Vermeidung einer Verunreinigung der Reaktionsprodukte sowie aus der Möglichkeit, kontinuierliche Reaktionen in Säulen oder ähnlichen Reaktoren durchführen zu können.

Es sind eine Reihe von Möglichkeiten der Immobilisierung biologisch aktiver Substanzen bekannt. Es wurde jedoch noch kein Verfahren zur Bindung oder Immobilisierung von biologisch aktiven Substanzen gefunden, welches weitestgehend frei von erheblichen Mängeln ist. Die adsorptive und die ionische Bindung befriedigen beispielsweise im allgemeinen nicht alle Ansprüche bezüglich der Festigkeit und Dauerhaftigkeit. Auch die Einbettung einer biologisch aktiven Substanz in einen polymeren Träger hat gewisse Nachteile. Die Polymerisation des Trägers wird dabei in Gegenwart der biologisch aktiven Substanz vorgenommen, wobei in der Regel ein Teil der biologischen Wirksamkeit verlorengeht.

Bei den gebräuchlichsten Verfahren zur Bindung einer biologischen aktiven Substanz erfolgt die Herstellung einer kovalenten Bindung zum Träger. Auch hierfür werden zahlreiche Verfahren beschrieben. Eine ausführliche Übersicht hierüber befindet sich z.B. in Methods in Enzymology, Vol. XLIV: Imobilized Enzymes, Academic Press, 1976. Auch dabei ist bekannt, dass die bisher verwendeten Träger zahlreiche Mängel aufweisen.

Träger auf der Basis von Polysacchariden, wie Cellulose, Stärke, Dextran, Agarose sowie von deren Derivaten, sind gegen bakteriellen oder enzymatischen Abbau nicht resistent, besitzen grösstenteils unbefriedigende hydrodynamische Eigenschaften und sind teilweise ausserordentlich teuer. Anorganische Träger, wie Aluminiumoxid, Silicagel, diverses keramisches Material oder poröses Glas, bereiten dagegen bei der Einstellung der Porendurchmesser und der Porenvolumen erhebliche Schwierigkeiten. Ausserdem ist bei den anorganischen Trägern das Anbringen von reaktiven, d.h. biologisch aktive Substanzen kovalent bindende Gruppen zum Teil sehr schwierig und aufwendig. Bislang verwendete polymere Träger kommen überwiegend in Form von Gelen (z.B. DE-OS 2 260 185, DE-OS 2 263 289) und Schäumen (z.B. DE-AS 1 642 596, DE-OS 2 365 854, DE-OS 2 612 138, DE-OS 2 625 471, DE-OS 2 625 544, US-PS 3 939 574) zur Anwendung und weisen ein unbefriedigendes hydrodynamisches Verhalten auf, d.h. dass die Durchflussgeschwindigkeiten begrenzt sind und sich aufgrund der unbefriedigenden mechanischen Eigenschaften nicht steigern lassen.

Polymere Träger mit Isocyanat- oder Aldehydgruppen als bindende Gruppen werden beispielsweise in der DE-OS 2 621 974 und DE-OS 1 915 970 beschrieben, wobei für die verwendeten polymeren Trägermaterialien auch die oben angegebenen Nachteile gelten.

Weiter sind in der FR-PS 2 396 030 synthetische Polymere aus Diaminen und Sulfochloriden des Polystyrols beschrieben. Diese Polymere sind jedoch Ionenaustauscher, die Enzyme nicht kovalent binden können.

Aus der GB-PS 1 252 336 sind Pfropfcopolymerisate bekannt, die Gruppen enthalten, welche mit Carbonylgruppen-haltigen Steroiden reagieren können.

In der FR-PS 2 242 111 wird auf Enzymträger auf Polystyrol-Basis hingewiesen [Makromol. Chem. 91, 136 (1966)], die jedoch keine befriedigende Ausbeuten liefern, wenn sie für enzymatische Umsetzungen eingesetzt werden. Ausserdem gibt diese FR-PS keinen Hinweis darauf, welche Polymerisate als Träger für Enzyme brauchbar sind.

Der Erfindung liegt die Aufgabe zugrunde, poröse organische polymere Träger zu schaffen, die die bekannten Nachteile vermeiden und die biologisch aktive Substanzen unter weitgehender Erhaltung ihrer Aktivität kovalent zu binden vermögen, so dass die bei der Umsetzung entstehenden biologisch aktiven Präparate unter den üblichen Bedingungen der Anwendung stabil sind. Die Präparate sollen eine hohe Aktivität besitzen und aufgrund ihrer hydrodynamischen Eigenschaften zum Betrieb in einer Säule geeignet sein.

Die Lösung der Aufgabe besteht in der Herstellung eines makroporösen vernetzten Styrolharzes als Trägermaterial zur kovalenten Bindung von Proteinen, das Isocyanat, Thioisocyanat oder Aldehydgruppen als proteinbindende Gruppen und gegebenenfalls als hydrophile Gruppen Sulfonsäuregruppen, gegebenenfalls in Form des Natriumsalzes, oder als Sulfonsäureamidgruppen enthält.

Das erfindungsgemässe Trägermaterial wird hergestellt aus einem sulfochlorierten makroporösen vernetzten Styrolharz, das 1 bis 4,5, bevorzugt 2 bis 4, Milliäquivalent (mÄ)/g Sulfonsäurechloridgruppen enthält und eine Porengrösse von $2 \cdot 10^{-5}$ bis $15 \cdot 10^{-5}$ mm, bevorzugt $5 \cdot 10^{-5}$ bis $13 \cdot 10^{-5}$ mm, aufweist. Die Herstellung derartiger Styrolharze ist dem Fachmann grundsätzlich bekannt und wird beispielsweise in der Literatur beschrieben von Brutskus et al. in Coll. J. USSR 34, 438–442 (1972).

Für die Herstellung des erfindungsgemäss zu

verwendenden Styrolharzes werden 97 bis 50, bevorzugt 80 bis 60, Gew.-% Styrol und 3 bis 50, bevorzugt 7 bis 25, Gew.-% Divinylbenzol und gegebenenfalls 1 bis 25 Gew.-% Äthylvinylbenzol verwendet, wobei gegebenenfalls, bezogen auf das Gesamtgewicht, zusätzlich 1 bis 10 Gew.-% eines Comonomeren aus der Gruppe 2- oder 4-Vinylpyridin, N-Vinylimidazol, N-Vinylpyrrolidon, Hydroxypropylacrylat oder tert.-Butylacrylat einpolymerisiert werden können, und die Polymerisation wird in an sich üblicher Weise durchgeführt.

Dabei wird das Suspensionspolymerisationsverfahren bevorzugt und in der Regel werden Perlen mit einem Durchmesser von 0,2 bis 1,5 mm erhalten. Von den oben angegebenen Comonomeren kommt das Äthylvinylbenzol als Comonomer insbesondere deshalb in Betracht, da technisches Divinylbenzol, falls dieses beispielsweise verwendet wird, 1 bis zu 50 Gew.-% Äthylvinylbenzol enthält, d.h. dass im erfindungsgemässen Trägermaterial bis zu 25% Äthylvinylbenzol als Comonomer enthalten sein können.

Bei der Polymerisation dienen als Porenbildner zweckmässigerweise Alkane mit 7 bis 12 C-Atomen, bevorzugt n-Octan oder ein üblicherweise erhältliches Alkangemisch, wie es beispielsweise ein höhersiedender Petroläther darstellt. Die Menge des verwendeten Porenbildners beträgt 50 bis 140, bevorzugt 80 bis 120 Gew.-%, bezogen auf das Gesamtgewicht der eingesetzten Monomeren.

Auch wenn Comonomere einpolymerisiert werden, werden Polymere mit den oben angegebenen Porengrössen erhalten. Entsprechend der angegebenen Porengrössen sind die Porenvolumina 0,5 bis 2 $cm^3/g$, bevorzugt 0,7 bis 1,5 $cm^3/g$, quecksilberporosimetrisch gemessen.

Das erfindungsgemäss zu verwendende makroporöse vernetzte Styrolharz wird anschliessend sulfochloriert. Die Sulfochlorierung erfolgt in an sich üblicher Weise analog der Umsetzung von monomeren Aromaten, wie sie beispielsweise in Houben-Weyl, Methoden der organischen Chemie, Band 9, Seiten 572 bis 578 beschrieben werden. Entsprechend den angewandten Bedingungen beträgt der Gehalt an Sulfonsäurechloridgruppen 1 bis 4,5 mÄ/g, bevorzugt 2 bis 4 mÄ/g.

Ausgehend von den Sulfonsäurechloridgruppen werden anschliessend die bindenden und gegebenenfalls hydrophilen Gruppen aufgebaut. Die bindenden Gruppen übernehmen die kovalente Bindung der Proteine, die hydrophilen Gruppen bewirken hydrophile Eigenschaften des Trägers und haben die Aufgabe, dem Träger entsprechend den verwendeten Proteinen sauren bis basischen Charakter zu verleihen.

In der Regel sollen die bindenden Gruppen ein Protein fixieren können, indem sie bei Temperaturen von 0 bis 50°C in wässriger Lösung mit primären Aminogruppen und/oder Hydroxylgruppen der Proteine reagieren und dabei eine kovalente Bindung zu den Sauerstoff- oder Stickstoffatomen der Hydroxyl- oder Aminogruppen der Proteine herstellen.

Gegenstand der Erfindung ist ein Verfahren zur Herstellung eines makroporösen polymeren Trägermaterials zur kovalenten Bindung von aktiven Proteinen über Isocyanat-, Thioisocyanat- oder Aldehydgruppen, das dadurch gekennzeichnet ist, dass man die Sulfonsäurechloridgruppen eines sulfochlorierten makroporösen vernetzten Styrolharzes, enthaltend 1 bis 4,5 mÄ/g Sulfonsäurechloridgruppen und bestehend aus Einheiten die sich ableiten von (I) 50 bis 97 Gew.-% Styrol (II) 3 bis 50 Gew.-% Divinylbenzol (III) gegebenenfalls 1 bis 25 Gew.-% Äthylvinylbenzol und (IV) gegebenenfalls 1 bis 10 Gew.-% eines weiteren Comonomeren, jeweils bezogen auf das Gesamtgewicht,

a) mit einer α, ω-Diaminoverbindung der Formel

$$H_2N-(CH_2)_n-NH_2,$$

in der n eine Zahl von 2 bis 12 bedeutet, oder Hydrazin

oder einem α,ω-Diaminodiäther der Formel

$$H_2N-(CH_2)_3-O-X-O-(CH_2)_3-NH_2,$$

in der $X-(CH_2)_m-$, wobei m eine Zahl von 2 bis 6 darstellt, oder den 2,2-Dimethyl-propylen-1,3- oder den 3-Methyl-pentylen-1,5-Rest bedeutet, umsetzt und

b) gegebenenfalls noch vorhandene Sulfonsäurechloridgruppen zur freien Sulfonsäure oder ihrem Natriumsalz verseift oder mit einem primären oder sekundären Amin zum Sulfonamid umsetzt und

c) anschliessend die endständigen Aminogruppen aus dem ersten Verfahrensschritt mit Phosgen, Thiophosgen,

oder einem aliphatischen, aromatischen oder cycloaliphatischen Diisocyanat bzw. Gemischen davon oder mit einem aliphatischen Dialdehyd der Formel

$$OCH-(CH_2)_p-CHO,$$

in der p die Zahlen 1 bis 8 bedeutet, zur Herstellung der bindenden Gruppen umsetzt.

Zum Aufbau der bindenden Gruppen werden im ersten Schritt 10 bis 100, bevorzugt 10 bis 80% und besonders bevorzugt 10 bis 50% der Sulfonsäurechloridgruppen des Trägermaterials mit einer α,ω-Diaminoverbindung der Formel

$$H_2N-(CH_2)_n-NH_2,$$

in der n die Zahlen 2 bis 12, bevorzugt 2 bis 8, bedeutet, Hydrazin oder mit einem α,ω-Diaminodiäther der Formel

$$H_2N-(CH_2)_3-O-X-O-(CH_2)_3-NH_2,$$

in der

$X-(CH_2)_m-$, wobei m die Zahlen 2 bis 6, bevorzugt 2 bis 4 darstellt,

oder den Rest

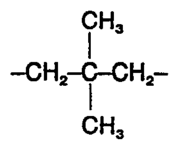

oder $-(CH_2)_2-CH(CH_3)-(CH_2)_2-$ bedeutet, umgesetzt.

Diese Umsetzung erfolgt in an sich üblicher Weise. Der Träger wird in wasserfreiem Dioxan oder Tetrahydrofuran mit einer α,ω-Diaminoverbindung oder Hydrazin 0,5 bis 5 Stunden auf Temperaturen von 50°C bis zur Siedetemperatur des Dioxans erhitzt. Dabei reagieren die Diaminoverbindungen überwiegend monofunktionell, so dass Seitengruppen mit endständigen Aminogruppen entstehen, die gleichzeitig den freiwerdenden Chlorwasserstoff binden. Die Menge der eingesetzten Diaminoverbindung ergibt sich aus dem gewünschten Umsetzungsgrad. Neben Dioxan und Tetrahydrofuran sind wegen der weiteren Aufarbeitung und weiteren Umsetzungen andere mit Wasser mischbare Lösungsmittel geeignet, sofern diese keine unerwünschten Nebenreaktionen mit den Sulfonsäurechloridgruppen eingehen. Auch aromatische, cycloaliphatische oder aliphatische Lösungsmittel, wie beispielsweise Toluol, Cyclohexan oder n-Octan können verwendet werden. Letztere sind jedoch weniger vorteilhaft, da sie nicht mit Wasser mischbar und deshalb nur schlecht aus den Poren des Trägermaterials zu entfernen sind.

Anschliessend werden gegebenenfalls noch vorhandene Sulfonsäurechloridgruppen in an sich üblicher Weise verseift zur freien Sulfonsäure oder bevorzugt zu deren Natriumsalz oder durch Umsetzung mit einem primären oder sekundären Amin in eine Sulfonamidgruppe überführt.

Die Verseifung zur Sulfonsäure oder deren Natriumsalz erfolgt zweckmässig durch Kochen in Wasser oder stark verdünnter, etwa 1- bis 4gewichtsprozentiger Natronlauge oder Natriumcarbonatlösung. Die Umsetzung zur Sulfonamidgruppe wird zweckmässigerweise in wasserfreiem Dioxan oder Tetrahydrofuran, bevorzugt in der Siedehitze, vorgenommen, wobei zur Bindung des freiwerdenden Chlorwasserstoffs das Amin im Überschuss verwendet wird.

Die Verseifung noch vorhandener Chlorsulfonsäuregruppen zur Sulfonsäure oder ihre Umsetzung zum Sulfonamid verleiht dem Trägermaterial in besonders vorteilhafter Weise saure, anionneutrale oder basische Eigenschaften. Zweckmässige primäre oder sekundäre Amine für die Überführung in ein Sulfonamid sind beispielsweise n-Butylamin, Di-n-propylamin, Diisopropylamin, Diisobutylamin, 1,2-Dimethylpropylamin, Diäthylamin, Monoäthanolamin, Diäthanolamin oder Dimethylaminopropylamin, wovon n-Butylamin, Di-n-propylamin und Dimethylaminopropylamin bevorzugt sind.

Im nächsten Schritt wird die Synthese der bindenden Gruppen fortgeführt, indem man die endständigen primären Aminogruppen mit Phosgen oder Thiophosgen zu Isocyanat oder Thioisocyanatgruppen oder mit einem aliphatischen, aromatischen oder Cycloaliphatischen Diisocyanat, zweckmässigerweise mit Hexan-1,6-diisocyanat, 4,4'-Diphenylmethandiisocyanat, Toluylendiisocyanat, Cyclohexan-1,4-diisocyanat, Dicyclohexylmethan-diisocyanat oder Methylcyclohexan-diisocyanat oder gegebenenfalls Mischungen hiervon, oder mit einem aliphatischen Dialdehyd der Formel

$$OHC-(CH_2)_p-CHO,$$

in der p die Zahlen 1 bis 8 bedeutet, bevorzugt Glutardialdehyd, umsetzt.

Die Umsetzung mit Phosgen oder Thiophosgen wird zweckmässigerweise in einem inerten Lösungsmittel, insbesondere in Toluol, in der Siedehitze durchgeführt. Die Umsetzung mit einem Diisocyanat wird zweckmässigerweise in wasserfreiem Dioxan oder Tetrahydrofuran bei Temperaturen von ca. 50°C zweckmässig in Gegenwart einer katalytischen Menge 1,4-Diaza-bicyclo(2,2,2)octan (DABCO) durchgeführt.

Das besonders bevorzugte Diisocyanat ist das Toluylendiisocyanat.

Bei der Umsetzung mit einem Dialdehyd wird der Träger in der wässrigen Lösung des Dialdehyds bei Raumtemperatur oder bevorzugt bei Temperaturen von 40 bis 100°C umgesetzt. Vorteilhafterweise werden die 2- bis 8fache molare Menge an Dialdehyd, bezogen auf die umzusetzenden Äquivalente an Aminogruppen, verwendet.

Bei der Herstellung der bindenden Isocyanat-, Thioisocyanat- oder Aldehydgruppen muss selbstverständlich dafür Sorge getragen werden, dass unerwünschte Nebenreaktionen ausgeschlossen sind. So ist beispielsweise von der Verwendung eines Diisocyanats für die Synthese der bindenden Gruppen abzusehen, wenn beispielsweise die hydrophile Gruppe durch Umsetzung mit einem Äthanolamin hergestellt worden ist, es sei denn, Nebenreaktionen werden billigend in Kauf genommen. Dagegen ist beispielsweise die Verwendung eines primären Amins in der hydrophilen Gruppe unbedenklich, wenn anschliessend eine Thiophosgenierung oder eine Umsetzung mit Diisocyanaten vorgenommen wird [vgl. J.Am. Chem. Soc. 68, 2506 (1946) oder Amn. Chem. 562, 214 (1949)].

Vorteilhafterweise hat ein erfindungsgemäss hergestelltes Trägermaterial im bevorzugten Bereich einen Gehalt an bindenden Gruppen von 0,2 bis 0,8 mÄ/g. Der optimale Bereich für eine besondere Aufgabe lässt sich gegebenenfalls leicht gesondert ermitteln.

Die Beladung des erfindungsgemässen Trägers mit einem Protein erfolgt, indem der Träger in die wässrige, einen Puffer enthaltende Lösung des zu bindenden Proteins bei Temperaturen von 0 bis 50°C eingebracht wird und man 30 Minuten bis 24 Stunden lang rührt. Die Beladung kann auch in der Weise erfolgen, dass man den Träger in einer Säule mit der pufferhaltigen Enzymlösung, gegebenenfalls im Kreislauf, durchströmen lässt.

Der beladene Träger wird in an sich üblicher Weise von der Lösung abgetrennt und kann beispielsweise in Form einer Säule einer Substratlösung ausgesetzt werden. Die bevorzugten Bedingungen zur Herstellung einer kovalenten Bindung mit einem Protein sind Temperaturen von 0

bis 8°C, pH-Werte von 2 bis 10 und Verweilzeiten der wässrigen Lösung von 1,5 bis 5 Stunden.

Als geeignete Puffer werden beispielsweise verwendet Phosphatpuffer, Acetatpuffer, Trishydroxymethylaminomethanpuffer, Äthanolaminpuffer, Triäthanolaminpuffer, Citratpuffer, Boratpuffer, Glycinpuffer, Glycylglycinpuffer, Alaninpuffer, Glycin-Hydrazin-Puffer, Natriumphosphat-Semicarbacid-Puffer.

Zur Beladung bzw. Immobilisierung auf dem Träger geeignete Enzyme sind vorzugsweise β-Fructosidase, Aminosäureacylase, Penicillinacylase und Glucoseisomerase.

Weiterhin können beispielsweise folgende biologisch aktive Proteine gebunden werden: Trypsin, Chymotrypsin, Pankreatin, α- und β-Amylase, Ribosenucleasen, Desoxyribonucleasen, Cellulase, Maltase, Pectinase, Chitinase, Pepsin, Bromelain, Keratinase, Amyloglucosidase, Lipase, Cholinesterase, Lecithinase, Phosphatase, Alginase, Asparaginase, Glutaminase, Urease, Lactase, Penicillinamidase, Glucoseoxidase, Katalase, Peroxidase, Lipoxidase, Xanthinoxidase, Cytochromreductase, Milchsäuredehydrogenase, Kollagenase, L-Aminosäureoxidase, D-Aminosäureoxidase, Rennin, Ficin, Subtilisin, Tannase, Phenyloxidase, Pullulanase, Pankreatin Isoamylase, Hexokinase, Galactoseoxidase, Diaphorase, Aldolase, Glykolsäureoxidase, Luciferase, Aldehydoxidase, Narringinase, Uricase, Glutathionreductase, Nitritoreductase, Nitratreductase, Bernsteinsäuredehydrogenase, Catecholoxidase.

Herstellung des Trägermaterials in der Sulfochlorid-Form.

Beispiel 1

Eine Suspension, bestehend aus 165 g Styrol, 135 g technischem Divinylbenzol (etwa 50% Äthylvinylbenzol enthaltend), 300 g n-Octan, 1,7 g Laurylperoxid, 1,3 g Polyvinylpyrrolidon als Suspensionshilfsmittel und 1,1 l Wasser, wird unter Stickstoff 7 Stunden bei 70°C und dann 2 Stunden bei 90°C gerührt. Das entstandene makroporöse perlförmige Polymerisat mit einem Perldurchmesser von 0,2 bis 1,5 mm wird in Methanol gewaschen und bei 70°C im Vakuum (<30 mbar) getrocknet. Ausbeute: 290,5 = 96,8% d.Th.

Zur Überführung in die Sulfochlorid-Form werden 113 g des erhaltenen Polymerisats in 450 ml Chloroform vorgelegt. Dann werden unter Rühren und Eiskühlung 300 ml Chlorsulfonsäure zugetropft. Anschliessend wird 2 Stunden bei Raumtemperatur, danach 6 Stunden in der Siedehitze gerührt. Das Produkt wird zuerst mit Chloroform und dann mit Dioxan gewaschen und bei 70°C im Vakuum getrocknet. Ausbeute: 230 g. Der Gehalt an Sulfonsäurechloridgruppen (durch Titration mit n/10 NaOH ermittelt) beträgt 3,8 mÄ/g.

Beispiel 2

4-Vinylpyridin enthaltendes Trägermaterial

Die Herstellung dieses Trägermaterials erfolgt analog Beispiel 1. Es werden 65 g Styrol, 30 g technisches Divinylbenzol, 5 g 4-Vinylpyridin, 100 g n-Octan, 3 g Laurylperoxid, 1,3 g Polyvinylpyrrolidon und 500 ml Wasser eingesetzt. Die Polymerisation wird während 7 Stunden bei 70°C durchgeführt. Ausbeute: 93,5 g = 93,5% d.Th. Es werden Perlen mit einem Durchmesser von 0,2 bis 1,5 mm erhalten.

Zur Überführung in die Sulfochlorid-Form werden analog Beispiel 1 40 g des erhaltenen Polymerisats mit 120 ml Chlorsulfonsäure in 313 g Chloroform umgesetzt. Ausbeute: 75 g. Der Gehalt an Sulfosäurechloridgruppen (durch Titration mit n/10 NaOH ermittelt) beträgt 4,11 mÄ/g.

Beispiel 3

Hydroxypropylacrylat enthaltendes Trägermaterial

Die Herstellung dieses Trägermaterials erfolgt analog zu Beispiel 1. Es werden 65 g Styrol, 30 g technisches Divinylbenzol, 5 g Hydroxypropylacrylat (Gemisch der 2-Hydroxy- und 3-Hydroxy-Isomeren), 100 g n-Octan, 3,5 g Laurylperoxid, 1,7 g Polyvinylpyrrolidon und 500 ml Wasser eingesetzt. Die Polymerisation wird während 7 Stunden bei 80°C durchgeführt. Ausbeute: 80,7 g = 80,7% d.Th. Es werden Perlen mit einem Durchmesser von 0,2 bis 1,5 mm erhalten.

Zur Überführung in die Sulfochlorid-Form werden 40 g des erhaltenen Polymerisats mit 120 ml Chlorsulfonsäure in 213 g Chloroform umgesetzt. Ausbeute: 67 g. Der Gehalt an Sulfonsäurechloridgruppen (durch Titration mit n/10 NaOH ermittelt) beträgt 4,0 mÄ/g.

Umsetzungen der Chlorsulfonsäuregruppen mit bindenden und hydrophilen Gruppen.

Beispiel 4

30 g Träger in der Sulfochlorid-Form (3,8 mÄ/g) entsprechend Beispiel 1 werden in 300 ml Dioxan mit 6,6 g Hexan-1,6-diamin unter Rühren 4 Stunden unter Rückfluss gekocht. Dabei werden 50% der Sulfonsäurechloridgruppen umgesetzt. Zum Verseifen der restlichen Sulfonsäurechloridgruppen wird das Polymer in Wasser übergeführt, mit Natriumbicarbonat neutralisiert und dann getrocknet.

Ausbeute: 26 g, wobei die freien Sulfonsäuren als Natriumsalz vorliegen.

25 g des erhaltenen Trägers werden 5 Stunden lang in 200 ml 25%iger wässriger Glutardialdehyd-Lösung unter Rückfluss gekocht. Dann wird 2 mal 1 Stunde lang bei Raumtemperatur in Wasser gerührt und anschliessend getrocknet.

Ausbeute: 30 g.

Beispiel 5

70 g Polymer in der Sulfochlorid-Form (3,7 mÄ/g) entsprechend Beispiel 1 werden in 200 ml Dioxan mit 13 g Hydrazinmonohydrat 4 Stunden bei 70°C gerührt. Dabei werden 50% der Sulfonsäurechloridgruppen umgesetzt. Zur Verseifung wird der Träger 2 Stunden lang in Wasser unter Rückfluss gekocht, mit Natriumbicarbonat neutralisiert, mit Wasser gewaschen und getrocknet.

Ausbeute: 65 g.

30 g des erhaltenen Trägers werden in 200 ml Dioxan mit 15,1 g Hexan-1,6-diisocyanat 1 Stunde bei Raumtemperatur gerührt. Dann werden 0,5 g DABCO zugesetzt und 6 Stunden bei 50°C gerührt. Anschliessend wird dreimal mit Dioxan gewaschen. Der Träger wird durch Absaugen grob vom Lösungsmittel befreit und dann lyophilisiert.

Ausbeute: 32 g.

Beispiel 6

25 g Polymer in der Sulfochlorid-Form (3,7 mÅ/g) entsprechend Beispiel 1 werden in 250 ml Dioxan mit 5,4 g Hexan-1,6-diamin unter Rühren 5 Stunden unter Rückfluss gekocht. Dabei werden 50% der Sulfochloridgruppen umgesetzt. Dann werden 10,1 g Di-n-propylamin zur Umsetzung der restlichen Sulfonsäurechloridgruppen (der Amin-Überschuss dient der Aufnahme des entstehenden Chlorwasserstoffs) zugesetzt und 5 Stunden bei 60°C gerührt. Anschliessend wird mit Methanol und Wasser gewaschen und getrocknet.

Ausbeute: 27 g.

25 g des erhaltenen Trägers werden mit 140 ml 25%iger wässriger Glutardialdehyd-Lösung und 130 ml Wasser 5 Stunden unter Rückfluss gekocht. Dann wird der Träger dreimal jeweils 1 Stunde bei Raumtemperatur in Wasser gerührt und danach getrocknet.

Ausbeute: 26 g.

Beispiel 7

70 g Polymer in der Sulfochlorid-Form (3,4 mÅ/g) gemäss Beispiel 1 werden in 300 ml Dioxan mit 27,8 g Hexan-1,6-diamin zur vollständigen Umsetzung der Sulfonsäurechloridgruppen 4 Stunden unter Rückfluss gekocht. Dann wird der Träger in Wasser 2 Stunden lang unter Rückfluss gekocht und anschliessend getrocknet.

Ausbeute: 67 g.

30 g des Trägers werden 10 Stunden in einer Lösung von 9,6 g Thiophosgen in 200 ml Toluol unter Rückfluss gekocht. Anschliessend wird 3 Stunden mit 200 ml Toluol und 9,6 g Pyridin unter Rückfluss gekocht. Danach wird nochmals mit Toluol und Dioxan gewaschen und lyophilisiert.

Ausbeute: 30,9 g.

Beispiel 8

30 g Polymer in der Sulfochlorid-Form (4,11 mÅ/g) gemäss Beispiel 2 werden in 250 ml Dioxan mit 3,6 g Äthylendiamin unter Rühren 4 Stunden unter Rückfluss gekocht. Dabei werden 50% der Sulfochloridgruppen umgesetzt. Dann werden 15,2 g Di-n-propylamin zugesetzt, wobei der Amin-Überschuss zur Aufnahme des entstehenden Chlorwasserstoffs dient, und 4 Stunden unter Rückfluss gekocht. Anschliessend wird mit Methanol und Wasser gewaschen und getrocknet.

Ausbeute: 29 g.

Der erhaltene Träger wird mit 250 ml Dioxan und 21,5 g Toluylendiisocyanat (Gemisch der 2,4- und 2,6-Isomeren) 1 Stunde bei Raumtemperatur gerührt. Dann werden 0,5 g DABCO zugesetzt und 6 Stunden bei 50°C gerührt. Anschliessend wird dreimal mit Dioxan gewaschen. Der Träger wird durch Absaugen grob vom Lösungsmittel befreit und dann lyophilisiert.

Ausbeute: 29 g.

Beispiel 9

30 g Polymer in der Sulfochlorid-Form (4,0 mÅ/g) gemäss Beispiel 3 werden in 250 ml Dioxan mit 14,7 g 4,9-Dioxadodecan-1,12-diamin unter Rühren 4 Stunden unter Rückfluss gekocht. Dabei werden 60% der Sulfochloridgruppen umgesetzt. Dann werden 11 g n-Butylamin zugesetzt, wobei der Amin-Überschuss der Aufnahme des entstehenden Chlorwasserstoffs dient, und 4 Stunden bei 60°C gerührt. Anschliessend wird mit Wasser gewaschen und getrocknet.

Ausbeute: 34 g.

30 g des erhaltenen Trägers werden mit 250 ml 16%iger wässriger Glutardialdehyd-Lösung 5 Stunden unter Rückfluss gekocht. Dann wird der Träger dreimal jeweils 1 Stunde bei Raumtemperatur in Wasser gerührt und dar h getrocknet.

Ausbeute: 37 g.

Beladung der Träger mit β-Fr osidase und Aminoacylase und Prüfung der gebundenen Enzymaktivität.

Beispiel 10

Zu einer Lösung von 450 mg β-Fructosidase oder Aminoacylase in 90 ml 0,02molarem Kaliumphosphat-Puffer (pH 8 bzw. 7,6) werden jeweils 3 g Träger entsprechend den Beispielen 4 bis 9 gegeben. Dann wird 2 Stunden unter Eiskühlung gerührt. Nach 1 Stunde und nach 2 Stunden werden die auf dem Träger gebundene Aktivität und die Restaktivität in der Lösung bestimmt.

Zur Prüfung der gebundenen β-Fructosidase-Aktivität in einer Säule (Durchmesser 1,5 bis 2 cm) wurde so vorgegangen, dass zunächst ein Nylon-Netz in die Säule eingebracht wurde. Darauf kam eine Schicht Kieselgel von 1 bis 2 cm Höhe und darauf der beladene Träger in einer Schichthöhe von 4 bis 5 cm. Die Durchflussrate der Substratlösung (Säulenvolumina/h = SV/h) wurde so eingestellt, dass die verfolgte Reaktion (Hydrolyse von Saccharose) zu weniger als 100% ablief. Als Substratlösung wurde eine 40%ige (Gew.-%) Saccharose-Lösung in 0,05molarem Natriumacetatpuffer (pH 4,65) verwendet. Der Umfang der Hydrolyse wurde polarimetrisch ermittelt.

Zur Prüfung der gebundenen Aminoacylase-Aktivität wurden 100 mg des beladenen Trägers 2 Minuten bei Raumtemperatur in 3 ml 0,1molarer Phosphat-Puffer-Lösung (pH 7,6), die 10 mg/ml N-Acetyl-D,L-Methionin und 0,36 mg Kobaltchlorid ($CoCl_2 \cdot 6\,H_2O$) enthielt, vorsichtig geschüttelt. Dann wurde die Menge des entstandenen L-Methionins nach dem Umsatz mit L-Aminosäureoxidase und Bestimmung des entstehenden Wasserstoffperoxids mit Peroxidase über eine UVspektralphotometrische Messung von o-Dianisidin (in der oxidierten, also chinoiden Form) ermittelt (Methods in Enzymology, Vol. II).

Die gebundenen Enzym-Aktivitäten sind der folgenden Tabelle zu entnehmen.

Trägergebundene Enzym-Aktivitäten

| Träger Beispiel Nr. | Aufbau der bindenden Gruppe | Hydrophile Gruppe | Enzym | Gebundene Enzymmenge [mg/g] | Aktivität | SV/h | Testdauer [d] Tage |
|---|---|---|---|---|---|---|---|
| 4 | Hexan-1,6-diamin/ Glutardialdehyd | $-SO_3Na$ (H) | β-Fructosidase | 142 | 90%[a] | 3,4 | 8 |
| 5 | Hydrazin/Hexan-1,6-diisocyanat | $-SO_3Na$ (H) | β-Fructosidase | 16,5 | 90%[a] | 1,1 | 7 |
| 6 | Hexan-1,6-diamin/ Glutardialdehyd | $-SO_2N$ $(Prop)_2$ | β-Fructosidase | 63 | 80%[a] | 2,4 | 6 |
| 7 | Hexan-1,6-diamin/ Thiophosgen | $-SO_2NH(CH_2)_6NH_2$ | β-Fructosidase | – | 80%[a] | 1,3 | 4 |
| 8 | Äthylendiamin/ Toluylendiisocyanat | $-SO_2N$ $(Prop)_2$ | β-Fructosidase | 31,9 | 62%[a] | 1,2 | 6 |
| 9 | 4,9-Dioxadodecan-1,12-diamin/ Glutardialdehyd | $-SO_2NH$ But. | β-Fructosidase | 12,2 | 58%[a] | 0,9 | 6 |
| 4 | s.o. | s.o. | Aminoacylase | 118,6 | 1,1 mg[b] | – | – |
| 5 | s.o. | s.o. | Aminoacylase | 136,6 | 1,2 mg[b] | – | – |

[a]: Hydrolyse in %, bezogen auf die eingesetzte Saccharose-Konzentration, wie im Text beschrieben.
[b]: Durch Hydrolyse gebildetes L-Methionin, wie im Text beschrieben.
SV/h: Säulenvolumina/Stunde.

**Patentansprüche**

1. Verfahren zur Herstellung eines makroporösen polymeren Trägermaterials zur kovalenten Bindung von aktiven Proteinen über Isocyanat-, Thioisocyanat- oder Aldehydgruppen, dadurch gekennzeichnet, dass man die Sulfonsäurechloridgruppen eines sulfochlorierten makroporösen vernetzten Styrolharzes, enthaltend 1 bis 4,5 mÄ/g Sulfonsäurechloridgruppen und bestehend aus Einheiten, die sich ableiten von (I) 50 bis 97 Gew.-% Styrol, (II) 3 bis 50 Gew.-% Divinylbenzol, (III) gegebenenfalls 1 bis 25 Gew.-% Äthylvinylbenzol und (IV) gegebenenfalls 1 bis 10 Gew.-% eines weiteren Comonomeren, jeweils bezogen auf das Gesamtgewicht,

a) mit einer α,ω-Diaminoverbindung der Formel

$$H_2N-(CH_2)_n-NH_2,$$

in der n eine Zahl von 2 bis 12 bedeutet, oder Hydrazin

oder einem α,ω-Diaminodiäther der Formel

$$H_2N-(CH_2)_3-O-X-O-(CH_2)_3-NH_2,$$

in der X $-(CH_2)_m-$, wobei m eine Zahl von 2 bis 6 darstellt, oder den 2,2-Dimethyl-propylen-1,3- oder den 3-Methyl-pentylen-1,5-Rest bedeutet, umsetzt und

b) gegebenenfalls noch vorhandene Sulfonsäurechloridgruppen zur freien Sulfonsäure oder ihrem Natriumsalz verseift oder mit einem primären oder sekundären Amin zum Sulfonamid umsetzt und

c) anschliessend die endständigen Aminogruppen aus dem ersten Verfahrensschritt mit Phosgen, Thiophosgen,

oder einem aliphatischen, aromatischen oder cycloaliphatischen Diisocyanat bzw. Gemischen davon oder mit einem aliphatischen Dialdehyd der Formel

$$OCH-(CH_2)_p-CHO,$$

in der p die Zahlen 1 bis 8 bedeutet, zur Herstellung der bindenden Gruppen umsetzt.

2. Verfahren nach Anspruch 1, dadurch gekennzeichnet, dass das sulfochlorierte makroporöse Styrolharz als weiteres Comonomeres (IV) 2- oder 4-Vinylpyridin, N-Vinylimidazol, N-Vinylpyrrolidon, Hydroxypropylacrylat oder tert.-Butylacrylat einpolymerisiert enthält.

3. Verfahren nach Anspruch 1 und/oder 2, dadurch gekennzeichnet, dass man in Stufe (a) eine α,ω-Diaminoverbindung der Formel

$$H_2N-(CH_2)_n-NH_2,$$

in der n eine Zahl von 2 bis 8 bedeutet, verwendet.

4. Verfahren nach Anspruch 1 und/oder 2, dadurch gekennzeichnet, dass man in Stufe (a) Äthylendiamin, Hexan-1,6-diamin oder 4,9-Dioxadodecan-1,12-diamin als α,ω-Diaminoverbindung verwendet.

5. Verfahren nach Ansprüchen 1 bis 4, dadurch gekennzeichnet, dass man in Stufe (b) mit Di-n-propylamin oder n-Butylamin umsetzt.

6. Verfahren nach Ansprüchen 1 bis 5, dadurch gekennzeichnet, dass man in Stufe (c) als Diisocyanat Hexan-1,6-diisocyanat, 4,4′-Diphenylmethandiisocyanat, Toluylendiisocyanat, Cyclohexan-1,4-diisocyanat, Dicyclohexylmethan-diisocyanat, Methylcyclohexan-diisocyanat oder Mischungen hiervon oder als aliphatischen Dialdehyd Glutardialdehyd verwendet.

**Claims**

1. A process for the preparation of a macroporous polymeric carrier for covalently binding active proteins via isocyanate, thioisocyanate or aldehyde groups, characterized in that the sulfonic acid chloride groups of a sulfochlorinated macroporous crosslinked styrene resin, containing 1–4,5 milliequivalents/g of sulfonic acid chloride groups and containing (I) from 50 to 97% by weigth of styrene units, (II) from 3 to 50% by weight of divinylbenzene units, (III) with or without 1 to 25% by weight of ethylvinylbenzene units and (IV) with or without 1 to 10% by weight of units of a further comonomer, each based on the total weight,

(a) are reacted with an α,ω-diamino compound of the formula

$$H_2N-(CH_2)_n-NH_2$$

where n is a number from 2 to 12, with hydrazine or with an α,ω-diamino-diether of the formula

$$H_2N-(CH_2)_3-O-X-O-(CH_2)_3-NH_2$$

where X is $-(CH_2)_m-$, m being a number from 2 to 6, or where X is a 2,2-dimethyl-1,3-propylene or 3-methyl-1,5-pentylene radical, and

(b) any sulfonic acid chloride groups still remaining are hydrolyzed to the free sulfonic acid or saponified to its sodium salt or reacted with a primary or secondary amine, to give sulfonamide groups, and

(c) then the terminal amino groups from the first process step are reacted with phosgene, thiophosgene, an aliphatic, aromatic or cycloaliphatic diisocyanate or an aliphatic dialdehyde of the formula

$$OCH-(CH_2)_p-CHO$$

where p is a number from 1 to 8, to prepare the binding groups.

2. A process as claimed in claim 1, characterized in that the sulfochlorinated macroporous styrene resin contains as further comonomer IV 2- or 4-vinylpyridine, N-vinylimidazole, N-vinylpyrrolidone, hydroxypropyl acrylate or tert.-butyl acrylate units.

3. A process as claimed in claim 1 and/or 2, characterized in that an α,ω-diamino compound of the formula

$$H_2N-(CH_2)_n-NH_2$$

where n is a number from 2 to 8, is used in step (a).

4. A process as claimed in claim 1 and/or 2, characterized in that ethylene-diamine, hexane-1,6-diamine or 4,9-dioxadodecane-1,12-diamine is used as the α,ω-diamino compound in step (a).

5. A process as claimed in claims 1 to 4, characterized in that di-n-propylamine or n-butylamine is used in step (b).

6. A process as claimed in claims 1 to 5, characterized in that in step (c) hexane-1,6-diisocyanate, 4,4'-diphenylmethane diisocyanate, toluylene diisocyanate, cyclohexane-1,4-diisocyanate, dicyclohexylmethane diisocyanate, methylcyclohexane diisocyanate or a mixture of these is used as diisocyanate, and glutarodialdehyde is used as aliphatic dialdehyde.

**Revendications**

1. Procédé de préparation d'un matériau support polymère, macroporeux, pour la liaison covalente de protéines actives, par l'intermédiaire de groupes isocyanate, thioisocyanate ou aldéhyde, caractérisé par le fait que l'on fait réagir les groupes chlorure d'acide sulfonique d'une résine styroléinique sulfochlorée macroporeuse et réticulée, contenant 1 à 4,5 m.équiv./g de groupes chlorure d'acide sulfonique et constituée de motifs qui dérivent de (I) 50 à 97% en poids de styrène, (II) 3 à 50% en poids de divinylbenzène, (III) éventuellement 1 à 25% en poids d'éthylvinylbenzène et (IV) éventuellement 1 à 10% en poids d'un autre comonomère, proportion rapportée au poids total,

a) avec un composé α,ω-diamino de formule

$$H_2N-(CH_2)_n-NH_2$$

dans laquelle n est un nombre de 2 à 12 ou de l'hydrazine,

ou un α,ω-diaminoéther de formule

$$H_2N-(CH_2)_3-O-X-O-(CH_2)_3-NH_2,$$

dans laquelle X représente $-(CH_2)_m-$, m est un nombre de 2 à 6, ou le reste 2,2-diméthylpropylène-1,3 ou le reste 3-méthyl-pentylène-1,5, et

b) éventuellement on saponifie les groupes chlorure d'acide sulfonique encore présents pour les transformer en acide sulfonique libre ou son sel de sodium, ou on les fait réagir avec une amine primaire ou secondaire pour les transformer en sulfonamide, et

c) enfin, pour obtenir les groupes de liaison, on fait réagir les groupes amino terminaux provenant du premier stade du procédé, avec du phosgène, thiophosgène, ou un diisocyanate aliphatique, aromatique ou cycloaliphatique, ou des mélanges de celui-ci, ou avec un dialdéhyde aliphatique de formule

$$OCH-(CH_2)_p-CHO,$$

dans laquelle p est un nombre de 1 à 8.

2. Procédé selon la revendication 1, caractérisé par le fait que la résine styroléinique sulfochlorée, macroporeuse contient en polymérisation, comme autre comonomère IV 2- ou 4-vinylpyridine, N-vinylimidazole, N-vinylpyrrolidone, hydroxypropylacrylate, ou tert.-butylacrylate.

3. Procédé selon la revendication 1 et/ou 2, caractérisé par le fait que l'on utilise au stade (a) un composé α,ω-diamino de formule

$$H_2N-(CH_2)_n-NH_2$$

dans laquelle n est un nombre de 2 à 8.

4. Procédé selon la revendication 1 et/ou 2, caractérisé par le fait que l'on utilise, comme composé α,ω-diamino, au stade (a), de l'éthylène-diamine, hexane-1,6-diamine ou 4,9-dioxa-dodécan-1,12-diamine.

5. Procédé selon les revendications 1 à 4, caractérisé par le fait qu'on fait réagir, au stade (b), avec de la di-n-propylamine ou n-butylamine.

6. Procédé selon les revendications 1 à 5, caractérisé par le fait que l'on utilise, comme diisocyanate, de l'hexan-1,6-diisocyanate, 4,4'-di-phénylméthane-diisocyanate, toluylène-diiso-cyanate, cyclohexan-1,4-diisocyanate, dicyclo-hexylméthane-diisocyanate, méthyl-cyclohexane-diisocyanate, ou comme dialdéhyde aliphatique, du glutaraldéhyde.